# EUROPÄISCHE PATENTSCHRIFT

(11) **EP 2 488 103 B1**
(45) Veröffentlichungstag und Bekanntmachung des Hinweises auf die Patenterteilung: **05.07.2017**
(21) Anmeldenummer: 10767920.1
(22) Anmeldetag: 03.09.2010
(51) Int. Cl.: A61B 5/155

(54) **LANZETTENKOPPLUNGSMECHANISMUS**
COUPLING MECHANISM FOR LANCETS
MÉCANISME POUR COUPLAGE D'UNE LANCETTE

(30) Priorität: 13.10.2009 EP 09012895
(43) Veröffentlichungstag der Anmeldung: 22.08.2012
(73) Patentinhaber: Roche Diabetes Care GmbH, 68305 Mannheim (DE); F. Hoffmann-La Roche AG, 4070 Basel (CH)
(72) Erfinder: LIST, Hans, 64754 Hesseneck-Kailbach (DE); FLUEGGE, Kai, 52068 Aachen (DE)
(74) Vertreter: Durm & Partner
(86) Internationale Anmeldenummer: PCT/EP2010/005416
(87) Internationale Veröffentlichungsnummer: WO 2011/044971

(56) Entgegenhaltungen:
- EP-A1- 1 790 288
- EP-A1- 1 880 671
- EP-A1- 1 997 429
- EP-A1- 2 039 293
- EP-A1- 2 042 098
- EP-A1- 2 050 392
- EP-A1- 2 113 197
- WO-A1-03/071940
- WO-A1-2004/064636
- WO-A1-2008/145628
- WO-A2-2004/060446
- WO-A2-2008/145625
- US-A1- 2004 102 803
- US-A1- 2007 191 738

## Beschreibung

Die Erfindung betrifft einen Lanzettenkopplungsmechanismus für einen Apparat zur Gewinnung und Analyse einer Blutprobe mit den im Oberbegriff des ersten Patentanspruchs angegebenen Merkmalen. Patienten mit Stoffwechselerkrankungen müssen regelmäßig ihr Blut analysieren. Speziell Diabetiker sind auf die regelmäßige Kontrolle des Blutzuckerspiegels angewiesen. Hierzu wird mittels einer Lanzette eine kleine Wunde erzeugt, vorzugsweise an einer Fingerkuppe. Von dem austretenden Blut wird dann eine kleine Probe gesammelt und auf ein Testelement übertragen, um anschließend analysiert zu werden.

In letzter Zeit wurden kleine, automatisch arbeitenden Handgeräte entwickelt, die ein Magazin mit einer Mehrzahl von als Einwegartikel (disposable) ausgebildeten Lanzetten enthalten sowie eine entsprechende Anzahl von Testelementen. Die Analyse der Blutprobe erfolgt mittels einer integrierten Messeinheit. Derart hoch integrierte Geräte haben den Vorteil, dass der Patient nur einen einzigen Apparat mit sich führen muss, mit dem er gleich eine Anzahl von Tests durchführen kann, bevor das Verbrauchsmaterial ausgetauscht werden muss.

Die konstruktiven Anforderungen an einen kleinen Handapparat, mit dem jedermann einen automatischen Blutzuckertest durchführen kann, sind äußerst anspruchsvoll und vielfältig: Das Gerät soll möglichst klein und leicht sein. Es muss sich so einfach und bequem bedienen lassen, dass ein Blutzuckertest überall und möglichst unauffällig durchgeführt werden kann. Absolute Zuverlässigkeit wird bei einem medizinischen Apparat selbstverständlich erwartet. Da Diabetes weit verbreitet ist, müssen die Fertigungskosten in einem für ein Massenprodukt vertretbaren Rahmen bleiben.

Für ein voll automatisch arbeitendes Blutzucker-Testgerät wird ein spezieller Antriebsmechanismus benötigt, der verschiedene und höchst unterschiedliche Bewegungen ausführen muss. Dazu gehören die schnelle Stechbewegung der Lanzette und die anschließende Rückzugsbewegung, die Fortschaltung des Magazins, um eine unverbrauchte Lanzette in Funktionsposition zu bringen, das Ankoppeln einer frischen Lanzette und das Abkoppeln der verbrauchten Lanzette sowie eine Kinematik, die eine Übertragung der Blutprobe von der Lanzette auf das Testelement bewirkt.

WO 2009/030340 A1 beschreibt ein Analysegerät zur Bestimmung eines Analyten in einer Körperflüssigkeit, umfassend ein trommelförmig ausgebildetes Magazin mit einer Mehrzahl von Stechelementen und einer Mehrzahl von Analyseelementen. Die Stechelemente haben einen Kapillarkanal und eine Probenübergabezone.

WO 2009/027010 A2 beschreibt ein Analysesystem mit einem rechteckigen Magazin, in dem eine Anzahl von Einweg-Lanzetten und Analyseelementen gespeichert sind.

Die ältere europäische Patentanmeldung 08010403.7 betrifft ein Analysesystem umfassend ein Magazin mit Kammern enthaltend Analyseelemente mit einer Probenkontaktzone und Stechelemente mit einem Kapillarkanal. Das Analysesystem umfasst ferner ein wiederverwendbares Analysegerät mit einem Stechantrieb, einer Halterung zur Aufnahme des Magazins sowie einer Mess- und Auswerteinrichtung.

Aus US 2004/0092995 A2 ist ein Apparat zur Gewinnung und Analyse einer Blutprobe bekannt, bei dem eine Anzahl von Einweg-Lanzetten in nebeneinander liegenden Kammern eines flachen Rundmagazins gespeichert sind. Ein Lanzettenantrieb ist selektiv an eine der Lanzetten ankoppelbar und bewegt diese in radialer Richtung. Die Vorrichtung enthält ferner eine Anzahl von Analyseelementen.

Das An- und Abkoppeln von Einmal-Lanzetten an einen Lanzettenantrieb ist in EP 1 333 756 B1 beschrieben. Die Lanzetten haben angespritzte flexible Ärmchen, die an einem Stößel mit Hinterschnitt angreifen.

Ein Lanzettenantrieb mit einem durch eine Antriebsfeder antreibbaren Antriebsrotor, einem Spannrotor und einem Lanzetten-Kopplungsmechanismus ist beispielsweise aus EP 1 669 028 A1 bekannt. Das Spannen der Antriebsfeder erfolgt durch Verdrehen des Spannrotors gegenüber dem Antriebsrotor über ein Getriebe, das von einem Elektromotor angetrieben wird. Der Lanzetten-Kopplungsmechanismus setzt die Rotationsbewegung des Antriebsrotors mittels einer Kurvensteuerung in eine Translationsbewegung der aktiven Lanzette um. Der Spannrotor und der Antriebsrotor können auch synchron bewegt werden, wenn sie durch einen Sperrriegel miteinander gekoppelt werden. Ausgehend von dem genannten Stand der Technik liegt der vorliegenden Erfindung die Aufgabe zugrunde, einen Lanzettenkopplungsmechanismus gemäß dem Oberbegriff des ersten Patentanspruchs besonders einfach auszubilden, bei gleichzeitig sehr hoher mechanischer Zuverlässigkeit und Geräuschlosigkeit im Betrieb.

Die genannte Aufgabe wird gelöst durch einen Lanzettenkopplungsmechanismus mit den im Patentanspruch 1 angegebenen Merkmalen.

Die Biegbarkeit der Lanzette in Verbindung mit der gekrümmt ausgebildeten Kammer führt dazu, dass die in der Kammer gelagerte Lanzette entsprechend der Krümmung der Kammer elastisch gebogen ist. Auf Grund der Biegespannung sitzt die Lanzette klemmend in der Kammer. Dadurch liegt die Lanzette mit Federkraft an der Wandung der Kammer an. Dies hat den Vorteil, dass die Lanzette in der Kammer trotz des unumgänglichen Spiels selbst dann in ihrer Position verbleibt, wenn die Kammer bewegt oder gar Stöße auf sie ausgeübt werden. Das vermeidet Klappergeräusche beim Transport und der Handhabung des Geräts. Vor allem aber kommt der erfindungsgemäße Koppelmechanismus ohne zusätzliche bewegte Teile aus, was ihn einfach in der Herstellung und extrem zuverlässig im Betrieb macht.

Wird die Lanzette entlang der Stichachse aus der gekrümmten Kammer herausgezogen, entspannt sie sich und nimmt wieder ihre ursprüngliche Form an. Die elastische Umformung der Lanzette beim Übergang vom gebogenen in den entspannten Zustand und umgekehrt wird dazu benutzt, die in einer zentralen Führungsnut geführte Treibstange an die Lanzette anzukoppeln. Nur wenn sich die Lanzette in gebogenem Zustand befindet, ist die Treibstange an die Lanzette ankoppelbar. Befindet sich die Lanzette dagegen in entspanntem Zustand außerhalb der Kammer, steht die Treibstange mit der Lanzette in formschlüssiger Verbindung. Es kann nun eine kontrollierte Stechbewegung entlang der Stichachse ausgeführt werden. Der Formschluss zwischen Treibstange und Lanzette erlaubt es auch, die Lanzette nach dem Stich wieder zurückzuziehen. Wird sie so weit zurückgezogen, dass sie wieder in den gekrümmten Schacht der Kammer einfährt, verbiegt sich die Lanzette erneut elastisch. Dadurch lässt sich die formschlüssige Verbindung zwischen Lanzette und Treibstange wieder lösen.

EP 1 997 429 beschreibt ein System zur Entnahme von Körperflüssigkeit mit einer flexiblen Lanzette, die in ihrer ursprünglichen Form länglich und gerade ist, und mit einem gebogenen Führungselement, das mit der Lanzette während des Stechvorgangs in Kontakt tritt und dabei die Lanzette in ihrer Bewegung führt, so dass diese in Bezug auf die Antriebsrichtung elastisch gebogen wird. Nach dem Kontakt mit dem Führungselement nimmt sie wieder ihre ursprüngliche Form an. Das gebogene Führungselement dient dazu, unter möglichst geringer Krafteinwirkung ein Abbiegen der Lanzettenspitze um 5 Grad bis 90 Grad von der Antriebsrichtung zu ermöglichen. Hierdurch ergibt sich der Vorteil, dass andere geometrische Anordnungen realisiert würden können als das mit einer rein axialen Antriebsrichtung für Lanzettenkörper und Lanzettenspitze möglich wäre. Zur Ankoppelung an eine Antriebseinheit besitzt die Lanzette eine Ankoppelstruktur, beispielsweise in Form von Nuten, Löchern, Kerben oder Ausstülpungen. Im Bereich der Ankopplungsstruktur soll die Lanzette gerade nicht abbiegbar, sondern stabil ausgefertigt sein, um den Kräften bei der Stechbewegung zu genügen. Der gebogene Teil der Lanzette im Bereich der Spitze dient nicht dem An- oder Abkoppeln einer Antriebseinheit wie zum Beispiel einer Treibstange.

WO 2004/060446 A2 offenbart in Fig. 51 einen Lanzettenkopplungsmechanismus mit einer hakenförmigen Koppelstruktur, jedoch weder eine Biegung der Lanzette noch eine Kammer mit gebogenem Schacht, der die Lanzette aufnimmt.

EP 1 790 288 A1 beschreibt eine Lanzette mit einem Knickbereich, der sich durch veränderte Steifigkeit auszeichnet. Dadurch kann die Spitze der Lanzette aus einer Trägerbandebene herausgeknickt werden. Dieses Abknicken hat mit dem An- und Abkoppeln der Lanzette an eine Treibstange oder einen Stößel nichts zu tun.

Die auf eine ältere Anmeldung derselben Anmelder zurückgehende WO 2010/130664 A1 beschreibt die Ankoppelung eines Stößels an ein Stechelement, das als Flachformteil ausgebildet ist und zwei Kopplungsarme mit Ösen aufweist. Die Kopplungsarme sind zur mechanischen Ankopplung an den Stechantrieb in einer vorgespannten Ausgangsstellung gehalten. Zu diesem Zweck weist das Magazin Führungsbahnen auf, die in einem proximalen Endabschnitt bogenförmig auseinanderlaufen und somit für eine entsprechende Aufspreizung der Kopplungsarme sorgen. Beim radialen Vorschub in Richtung des distalen Führungsbahnendes schwenken die Kopplungsarme in eine gemeinsame Führungsebene ein, in der die Ösen in den Stechantrieb selbsttätig einhaken, währen die Vorspannung aufgehoben ist. Der Antriebsstößel ist an einem Schlitten befestigt, der hin und her verschiebbar ist, um die Stechbewegung zu übertragen. Die Kammern, welche die Testeinheit mit dem Stechelement aufnehmen, haben keine Führung für den Antriebsstößel.

Zur Herstellung der formschlüssigen Verbindung in entspanntem Zustand weist die Lanzette vorzugsweise eine Ankoppelausnehmung auf und weist die Treibstange an ihrem vorderen Ende eine Kopplungsstruktur auf, die sich senkrecht zur Biegeachse der Lanzette erstreckt und in die Auskoppelausnehmung der gebogenen Lanzette eingreifen kann. Wird die Lanzette aus der Kammer herausgezogen, bewegt sich die Ankoppelausnehmung auf einer Kreisbahn um die Biegeachse. Die Kopplungsstruktur bewegt sich dagegen nur in Richtung der Stichachse. Bei synchroner Bewegung von Treibstange und Lanzette in Richtung der Stichachse entsteht eine Relativbewegung zwischen der Ankoppelausnehmung und der Kopplungsstruktur in einer Richtung senkrecht zur Stichachse. Dadurch kommt die Auskoppelausnehmung automatisch in Eingriff mit der Kopplungsstruktur.

Die Lanzette kann einfach aus einem Stück ebenen flachem Blech gefertigt sein. In ihrem hinteren Bereich kann sie eine Öse aufweisen. Die Treibstange kann an ihrem vorderen Ende einen korrespondierend ausgebildeten Haken aufweisen, der in die Öse einhakbar ist. Die Lanzetten, von denen ja große Mengen benötigt werden, lassen sich so sehr einfach und billig herstellen, beispielsweise durch Stanzen. Es muss lediglich ein ausreichend elastisches Material, vorzugsweise Stahlblech, gewählt werden. Die Öse muss nur so breit sein, dass der Haken am vorderen Ende der Treibstange einhakbar ist. Es genügt, wenn die Lanzetten etwa 1 mm breit sind. Entsprechend schmal kann die Kammer zur Aufnahme der Lanzette ausgebildet sein, was der Forderung nach kleinen Abmessungen besonders entgegenkommt.

Es lässt sich eine Mehrzahl von Kammern nebeneinander in einem kreisförmigen Magazin anordnen, wobei sich die Schächte der Kammern in radialer Richtung erstrecken. Durch Verdrehen des Magazins lässt sich eine der Kammern in eine Position bringen, in der die Treibstange in die Kammer eindringen und an die in der Kammer befindliche Lanzette ankoppeln kann. Eine solche Anordnung der Kammern in einem flachen Ringmagazin erlaubt den Bau eines sehr kompakten Handgeräts mit niedriger Bauhöhe, vor allem wenn ein Rotorantrieb koaxial in der Mitte des Ringmagazins angeordnet wird. Das Magazin kann aus einem Unterteil und einem Oberteil bestehen, die in zusammengesetztem Zustand die Kammern bilden. Die Lanzetten lassen sich so in entspanntem Zustand in die noch offenen Kammern einlegen. Sobald das Oberteil aufgesetzt wird, werden die Lanzetten in die Krümmungen der entstehenden Schächte gezwungen und können ohne Kraft von außen nicht mehr aus ihrer vorgegebenen Position innerhalb des Magazins verrücken. Fertigungstechnisch besonders vorteilhaft ist, dass in einem Arbeitsgang alle Kammern des Magazins gleichzeitig mit Lanzetten gefüllt werden können und sich die für die Funktion des Lanzettenkopplungsmechanismus notwendige Biegung der Lanzetten durch einfaches Aufdrücken des Magazinoberteils erzeugt wird.

Weitere Einzelheiten, Funktionen und Wirkungen der erfindungsgemäßen Konstruktion werden anhand der beigefügten Abbildungen näher beschrieben. Es zeigen:
- Fig. 1: einen Apparat zur Gewinnung und Analyse einer Blutprobe, in vereinfachter perspektivischer Darstellung;
- Fig. 2: die Antriebseinheit des Apparats von Fig. 1, perspektivisch in größerem Maßstab;
- Fig. 3a: einen Blick von unten auf den Spannrotor der Antriebseinheit von Fig. 2;
- Fig. 3b: einen Blick von oben auf den Spannrotor;
- Fig. 4: den Spannrotor, von oben;
- Fig. 5a: den Antriebsrotor der Antriebseinheit von Fig. 2a, von oben;
- Fig. 5b: den Antriebsrotor, von unten;
- Fig. 6: einen Teil des Apparats von Fig. 1 mit eingesetztem Magazin, in einer vergrößerten perspektivischen Darstellung mit Vertikalschnitt in radialer Richtung durch die Drehachse;
- Fig. 7a: das Magazin des Apparats von Fig. 1 mit Lanzette, in vereinfachter perspektivischer Darstellung mit Längsschnitt durch eine Kammer;
- Fig. 7b: das Magazin gemäß Fig. 7a mit Lanzette und Treibstange der Antriebseinheit von Fig. 2;
- Fig. 8: die Treibstange von Fig. 7b, in stark vergrößerter perspektivischer Ansicht;
- Fig. 9a-9d: den Vorgang des Ankoppelns der Treibstange an eine Lanzette, schematisch;
- Fig. 10: eine alternative Ausführung einer Lanzette;
- Fig. 11: eine alternative Ausführung eines Lanzettenkopplungsmechanismus.

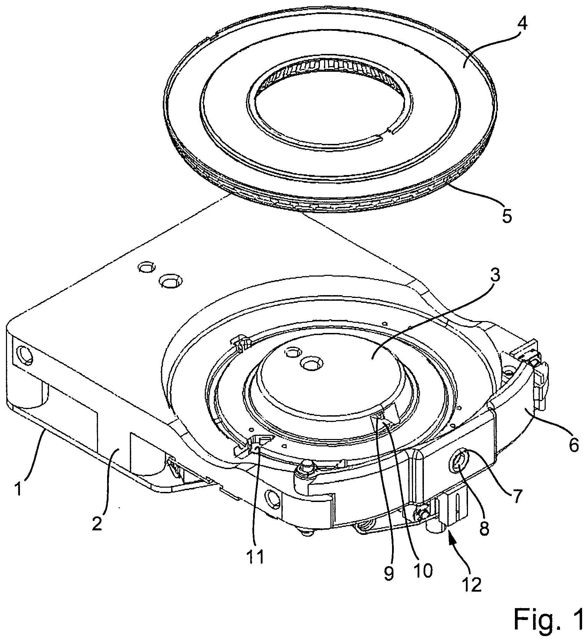

Tragendes Element des in Figur 1 dargestellten Apparats ist eine Grundplatte 1. Das Gehäuse umfasst eine obere Abdeckung 2, die auf die Grundplatte 1 montiert ist. Die übrigen Teile des Gehäuses sind in dieser Abbildung weggelassen. An der Oberseite ist eine runde Aufnahme 3 vorgesehen, auf die ein kreisringförmig ausgebildetes Magazin 4 aufgesetzt werden kann. Dieses Magazin 4 enthält eine Vielzahl von nebeneinander angeordneten, sich in radialer Richtung erstreckenden Kammern 5, in denen jeweils eine Lanzette gelagert ist. Das Magazin 4 enthält auch eine Vielzahl von Testelementen, die den Kammern 5 zugeordnet sind.

Die Stirnseite des Gehäuses wird gebildet von einem Andruckbügel 6. Etwa in der Mitte ist ein Fixierring 7 ausgebildet, der eine Öffnung 8 umschließt. Der Fixierring 7 dient zum Andrücken eines Fingers, aus dem eine Blutprobe entnommen werden soll. Dabei ragt die Fingerkuppe ein Stück in die Öffnung 8 hinein. Eine der im Magazin 4 gelagerten Lanzetten kann dann von hinten durch die Öffnung 8 in die Fingerkuppe eingestochen und wieder zurückgezogen werden, um eine Probe des aus der Stichwunde austretenden Bluts aufzunehmen.

Der Lanzettenantrieb ist unter der Abdeckung 2 verborgen. Zu sehen ist hier lediglich das vordere Ende einer Treibstange 9, die durch eine rechteckige Austrittsöffnung 10 in der Abdeckung 2 hindurchschaut. Im Betrieb, wenn das Magazin 4 auf der Aufnahme 3 sitzt, kann die Treibstange 9 aus der Öffnung 10 herausschnellen und von hinten in eine Kammer 5 des Magazins 4 eindringen, um die dort gelagerte Lanzette nach vorne in Richtung der Öffnung 8 zu treiben und anschließend wieder entlang der Stichachse zurück in die Kammer 5 hineinzuziehen. Die entnommene Blutprobe kann dann auf ein Testelement übertragen werden, um sie zu analysieren.

Die im Magazin 4 gespeicherten Lanzetten und Testelemente sind nur zum einmaligen Gebrauch bestimmt. Nach Gewinnung und Analyse einer Blutprobe wird deshalb das Magazin 4 um seine Achse gedreht, um so eine frische Lanzette in Funktionsposition zu bringen. Die hierzu benötigte Einrichtung zur Fortschaltung des Magazins 4 befindet sich unter der Abdeckung 2. Hier ist davon nur ein Kulissenschieber 11 zu sehen, welcher mit an der Unterseite des Magazins 4 vorgesehenen Zapfen zusammenarbeitet, um eine Bewegung des Kulissenschiebers 11 in radialer Richtung in eine Drehung des Magazins 4 um dessen Drehachse umzusetzen.

Unter dem Andruckbügel 6 schaut eine Einrichtung 12 hervor, welche eine Probenübertragungsbewegung senkrecht zur Stichachse erzeugt. Diese dient der Übertragung der von der Lanzette aufgenommenen Blutprobe auf ein zugeordnetes Testelement im Magazin 4, indem die Lanzette und das Testelement gegeneinander gedrückt werden.

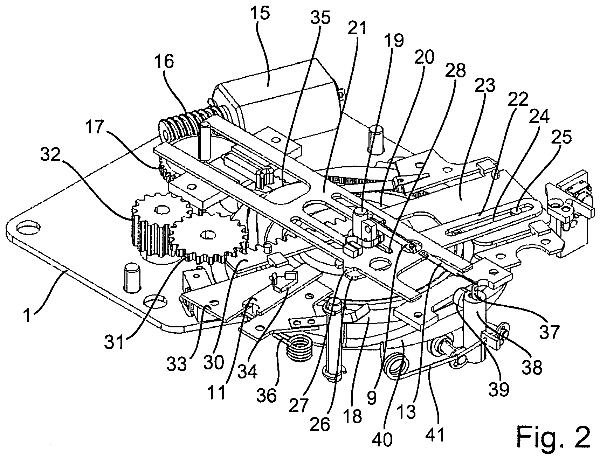

In Figur 2 sind die Details der Antriebseinheit zu sehen. Eine Lanzette 13 befindet sich in Funktionsposition vor der Treibstange 9.

Auf der Grundplatte 1 ist ein Elektromotor 15 befestigt. Dieser wird von einer Batterie mit Strom versorgt. Auf der Welle des Elektromotors 15 sitzt eine Schneckenwelle 16, die ein Schneckenrad 17 kämmt. Dadurch wird die Drehzahl des Elektromotors 15 stark untersetzt. Weitere Zahnräder, die sich teilweise unterhalb der Grundplatte 1 befinden, übertragen die Antriebskraft des Elektromotors 15 auf einen Spannrotor 18, der um eine senkrechte Drehachse 19 auf der Grundplatte 1 drehbar gelagert ist. Um dieselbe Drehachse 19 und damit koaxial zum Spannrotor 18 ist ein Antriebsrotor 20 drehbar gelagert. Spannrotor 18 und Antriebsrotor 20 sind über eine Spiralfeder drehbeweglich verbunden. Aus Gründen der Übersicht ist diese Spiralfeder, die als Antriebsfeder des Lanzettenantriebs dient, nicht eingezeichnet.

Wird nun mittels des Elektromotors 15 der Spannrotor 18 in Drehung versetzt, während der koaxiale Antriebsrotor 20 still steht, so wird die Antriebsfeder gespannt. Wird der Antriebsrotor 20 dann losgelassen, eilt er unter der Wirkung der sich entspannenden Antriebsfeder dem Spannrotor 18 hinterher. Diese schnelle Rotation des Antriebsrotors 20 wird mittels einer Kurvensteuerung in eine radiale Vor- und Rückbewegung eines Stechschlittens 21 umgesetzt. Der Stechschlitten 21 trägt eine Treibstange 9, die an eine Lanzette ankoppelbar ist.

Um den Hub der Lanzette zu variieren, wird die Rotation des Antriebsrotors 20 nicht direkt in eine radiale Vor- und Rückbewegung der Lanzette entlang der Stichachse umgesetzt. Die Umsetzung erfolgt vielmehr über einen einarmigen Übersetzungshebel 22, dessen Hebelachse auf einem quer zur Stichachse verfahrbaren Hebelschlitten 23 gelagert ist. Hierzu weist der Übersetzungshebel 22 ein Langloch 24 auf, in das ein Drehzapfen 25 des Hebelschlittens 23 eingreift. An seinem gegenüberliegenden freien Ende ist der Übersetzungshebel 22 als Gabel 26 ausgebildet, die einen am Rand des Stechschlittens 21 vorgesehenen Zapfen 27 umgreift. Eine Verschwenkung des Übersetzungshebels 23 um den Drehzapfen 25 führt damit zu einer linearen Bewegung des Stechschlittens 21 entlang der Stichachse bzw. in radialer Richtung, bezogen auf die Drehachse 19 des Antriebsrotors 20.

Zwischen dem Langloch 24 und der Gabel 26 trägt der Übersetzungshebel 22 einen Nutenreiter 28, der nach unten weist und in eine Steuernut 29 eingreift, welche in der Oberseite des Antriebsrotors 20 vorgesehen ist. Wird der Hebelschlitten 23 z. B. nach links verfahren, so wandert der Drehzapfen 25 im Langloch 24 nach rechts. Dadurch verlängert sich der Hebelarm zwischen Drehzapfen 25 und Nutenreiter 28 und damit das Übersetzungsverhältnis, mit dem eine radiale Bewegung des Nutenreiters 28 über den Übersetzungshebel 22 auf den Zapfen 27 des Stechschlittens 21 übertragen wird. Da der Hebelschlitten 23 parallel zur Ruhelage des Übersetzungshebels 22 verschoben wird, ändert sich nur der Hub des freien Hebelendes, das den Stechschlitten 21 antreibt, wohingegen die Ruhelage des Stechschlittens 21 nicht verändert wird. Dies erlaubt es, den Hub der Lanzette zu variieren, um die Stechtiefe anzupassen.

Die rückwärtige Kante des Hebelschlittens 23 ist als Zahnstange 30 ausgebildet, in die ein Zahnrad 31 eingreift. Der Antrieb erfolgt durch ein Zahnritzel 32. Damit kann der Hebelschlitten 23 nach rechts oder links verfahren werden, um das Übersetzungsverhältnis des Übersetzungshebels 23 einzustellen.

Die Antriebseinheit integriert ein Schrittschaltwerk mit einer zweiten Kurvensteuerung, welche die Rotationsbewegung des Spannrotors 18 in eine lineare Bewegung zum Fortschalten des Magazins 4 umsetzt. Das Schrittschaltwerk umfasst den Kulissenschieber 11, der auf einer Führung 33 in radialer Richtung beweglich gelagert ist. Der Kulissenschieber 32 trägt an seiner Oberseite eine Schaltkulisse 34. An der Unterseite des Magazins 4 sind entsprechend ausgeformte Schaltzapfen vorgesehen, die in die Schaltkulisse 34 von oben eingreifen, um die radiale Bewegung des Kulissenschiebers 32 in eine begrenzte Drehbewegung des Magazins 4 umzusetzen. Der Spannrotor 18 trägt an seiner Außenseite eine Schaltnocke 35. Ein Federbügel 36 drückt ein Betätigungselement des Kulissenschiebers 32 gegen die Außenseite des Spannrotors 18. Beim Überfahren der Schaltnocke 35 folgt der Kulissenschieber 11, der von der Schaltnocke 35 gebildeten Kurvenbahn, so dass die Rotationsbewegung des Spannrotors 18 in eine lineare Vor- und Rückbewegung des Kulissenschiebers umgesetzt wird. Diese kurzhubige Bewegung wird mittels der Schaltkulisse 34 auf das Magazin 4 übertragen, um dieses einen Schritt weiter zu drehen. Damit gelangt die nächste Kammer 5 mit einer neuen Lanzette in Funktionsposition.

Die Antriebseinheit umfasst ferner eine Einrichtung zur Erzeugung einer Probenübertragungsbewegung senkrecht zur Stichachse. In geringem Abstand vom Rand des Spannrotors 18 ist ein Druckstößel 37 in einer Gleithülse 38 vertikal beweglich gelagert. Der Druckstößel 37 ist mit einer Gleitrolle 39 verbunden. Am Spannrotor 18 ist im Bereich des äußeren Rands eine Rampe 40 ausgebildet, deren Oberseite von der Gleitrolle 39 abgefahren wird. Wird der Spannrotor 18, angetrieben vom Elektromotor 15, in langsame Drehung versetzt, so gelangt die Gleitrolle 39 bei einer bestimmten Winkelstellung des Spannrotors 18 in den Bereich der Rampe 40 und beginnt, sich nach oben zu verlagern. Dadurch wird der Druckstößel 37 nach oben bewegt. Nach Erreichen des Scheitelpunktes der Rampe 40 bewegen sich die Gleitrolle 39 und der Druckstößel 37 wieder nach unten.

Die von dem Druckstößel 37 ausgeführte Bewegung verläuft hier senkrecht zur Stichachse, kann aber auch eine Bewegungskomponente parallel zur Stichachse umfassen. Die Bewegung des Druckstößels 37 wird dazu benutzt, die aktive Lanzette nach dem Stich gegen ein Testelement zu drücken, um die aufgenommene Blutprobe zu übertragen. Anschließend zieht sich der Druckstößel 37 wieder zurück, wodurch sich die Lanzette wieder vom Testelement löst. Ein Federbügel 41 sorgt dafür, dass die Gleitrolle 39 gegen die Oberseite der Rampe 40 anfedert, so dass gewährleistet ist, dass die Gleitrolle 39 genau der Kontur der Rampe 40 folgt.

Die beschriebene Antriebseinheit generiert aus der Bewegung einer gemeinsamen zentralen Antriebsquelle, nämlich dem Elektromotor 15, alle für die Gewinnung und Analyse einer Blutprobe erforderlichen vielfältigen Bewegungen. Die Kraft des Elektromotors 15 wird durch einen Y-förmig verzweigten Antriebsstrang auf die in Funktionsposition befindliche Lanzette, das Magazin 4 und die Einrichtung zur Erzeugung einer Probenübertragungsbewegung senkrecht zur Stichachse übertragen. Zentrales Element dieses Antriebskraftübertragungsgetriebes ist der Spannrotor 18, der in Abhängigkeit von seinem Drehwinkel die Kraft des Elektromotors 15 selektiv verteilt, nämlich zum Spannen der Antriebsfeder des Lanzettenantriebs, zur Betätigung des Kulissenschiebers 32 oder zum Anheben des Druckstößels 37.

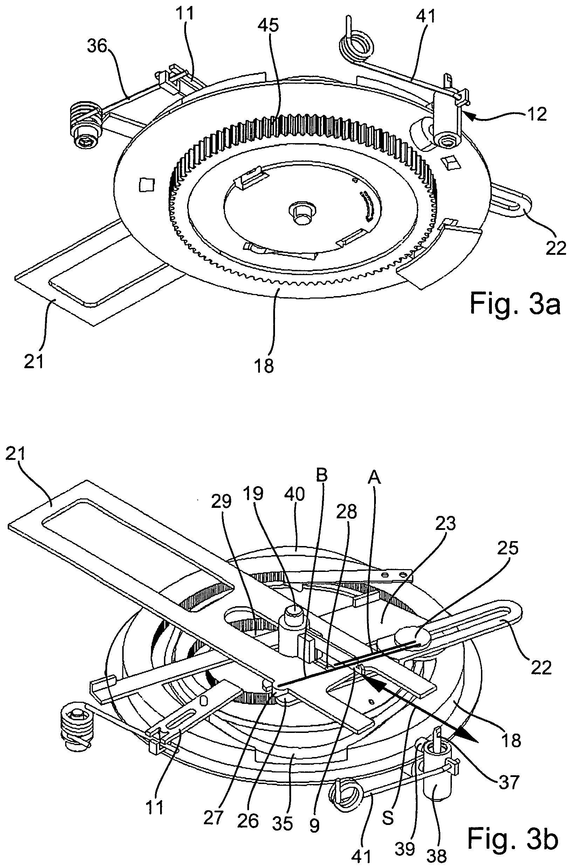

In den Figuren 3a und 3b ist die zuvor beschriebene Antriebseinheit zum besseren Verständnis auf die wesentlichen bewegten Elemente reduziert dargestellt. In der Mitte sitzt die gemeinsame Drehachse 19, um die der Spannrotor 18 und der Antriebsrotor 20 rotieren. Der Spannrotor 18 weist an seiner Unterseite (vgl. Figur 3a) einen Zahnkranz 45 auf, über den die Antriebskraft des Elektromotors 15 auf den Spannrotor 18 übertragen wird. Das in den Zahnkranz 45 eingreifende Zahnrad ist in dieser Abbildung ebenso fortgelassen wie die übrigen Elemente des Antriebskraftübertragungsgetriebes, das den Spannrotor 18 mit dem Elektromotor 15 kraftschlüssig verbindet.

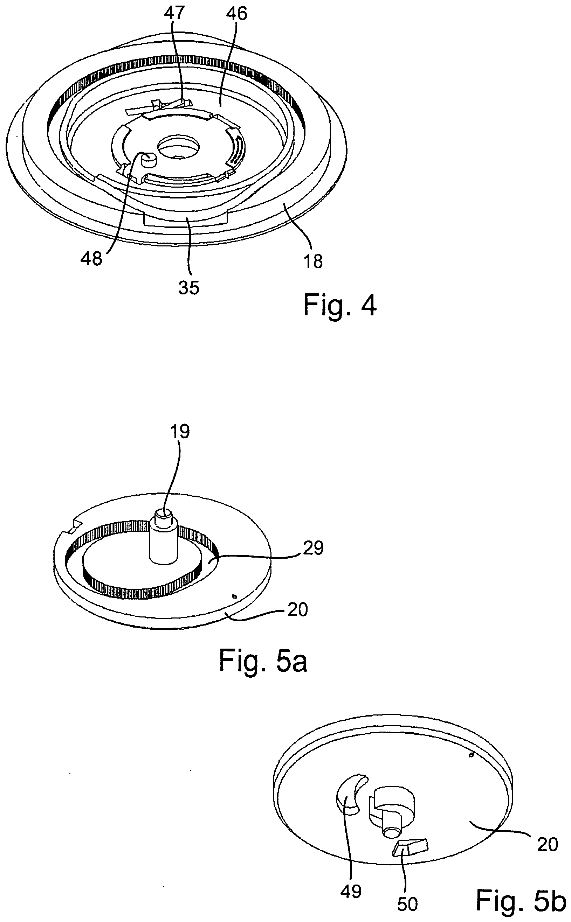

Der Spannrotor 18 hat die Grundform einer flachen Scheibe und weist eine zentrale topfartige Ausnehmung 46 auf, wie am besten in Figur 4 zu sehen ist. In dieser Ausnehmung 46 sitzt der Antriebsrotor 20, der als flache kreisförmige Scheibe ausgebildet ist, wie dies am besten in den Figuren 5a und 5b zu sehen ist. Am Boden der topfartigen Ausnehmung 46 des Spannrotors 18 ist eine Sperrklinke 47, die als Wippe ausgebildet ist, schwenkbar gelagert. In einigem Abstand davon sitzt ein Sperrzapfen 48. Dazu korrespondierend sind in der Unterseite des Antriebsrotors 20 eine gebogene Nut 49 für den Eingriff des Sperrzapfens 48 und eine Aussparung 50 vorgesehen, in welche die Sperrklinke 47 eingreifen kann. Dadurch lässt sich der Spannrotor 18 und der Antriebsrotor 20 wahlweise in einer Drehrichtung drehfest miteinander verbinden oder auch starr miteinander verklinken. Sind Spannrotor 18 und Antriebsrotor 20 mittels Sperrklinke 47 und Sperrzapfen 48 drehfest miteinander verbunden, so überträgt sich eine Rotationsbewegung des Spannrotors 18 unmittelbar auf den Antriebsrotor 20, so dass dieser auch langsame Bewegungen ausführen kann, wie sie insbesondere vor und nach einem Stich benötigt werden.

Figur 3b verdeutlicht auch nochmals die Funktion des Übersetzungshebels 22, dessen Nutenreiter 28 die Steuernut 29 des Antriebsrotors 20 abfährt (vgl. 5a). Der Übersetzungshebel 22 ist mittels des Drehzapfens 25 auf dem Hebelschlitten 23 gelagert, wobei das Langloch 24 ein Verfahren des Hebelschlittens 23 quer zur Stichachse S zulässt. Der Stechschlitten 21 mit der darauf montierten Treibstange 9 greift mit seinem Zapfen 27 in die Gabel 26 des Übersetzungshebels 22 ein. Das durch Verfahren des Hebelschlittens 23 einstellbare Übersetzungsverhältnis ergibt sich aus dem Verhältnis zwischen kurzem Hebelarm A und langem Hebelarm B, wobei der kurze Hebelarm A dem Abstand zwischen Nutenreiter 28 und Drehachse des Drehzapfens 25 entspricht und der lange Hebelarm B dem Abstand zwischen Drehzapfen 25 und Angriffspunkt der Gabel 26 am Zapfen 27 entspricht.

Die Umsetzung der Rotationsbewegung des Spannrotors 18 in eine zweite lineare Bewegung, die zur Fortschaltung des Magazins 4 (vgl. Figur 2) eingesetzt wird, geschieht mittels einer Schaltnocke 35, die bei der in Figur 3b dargestellten Winkelstellung des Spannrotors 18 gut sichtbar ist. Die Schaltnocke 35 wird von einem Gleitzapfen 51 abgefahren, der an der Unterseite des Kulissenschiebers 32 vorgesehen ist. Dabei sorgt der Federbügel 36 für eine federnde Anlage des Gleitzapfens 51 an die Randseite des Spannrotors 18. Der äußere Rand des Spannrotors 18 ist als flacher, nach außen weisender Flansch in der Art einer Hutkrempe ausgebildet. Dieser Rand verdickt sich im Bereich der Rampe 40 auf ein Mehrfaches. Wird die Rampe 40 von der Gleitrolle 39 abgefahren, so bewegt sich der in der Gleithülse 38 gelagerte Druckstößel 37 senkrecht nach oben, um eine Probenübertragungsbewegung auszuführen. Der Federbügel 41 sorgt für einen gleich bleibenden Anpressdruck, mit dem die Gleitrolle 39 auf den Rand des Spannrotors 18, insbesondere beim Überfahren der Rampe 40 drückt.

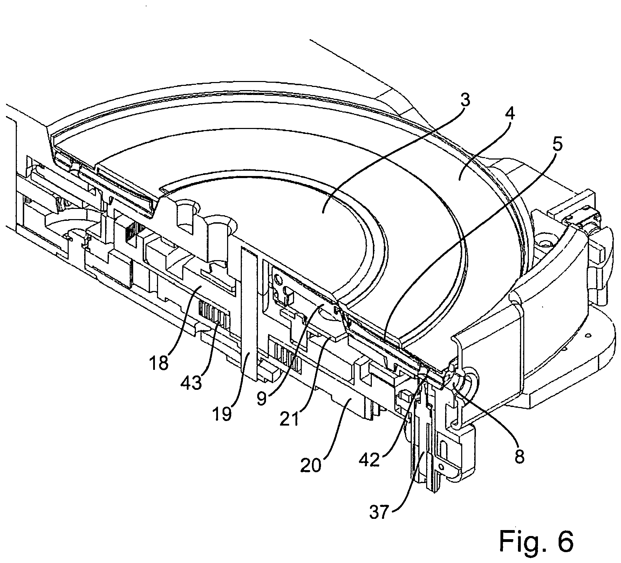

In Figur 6 sitzt das Magazin 4 auf der Aufnahme 3. Zu erkennen ist die gemeinsame Drehachse 19, um die der Spannrotor 18, der Antriebsrotor 20 und das Magazin 4 rotiert.

Der Vertikalschnitt von Figur 6 geht mitten durch eine der ringförmig angeordneten Kammern 5, die zur Aufnahme je einer Einweg-Lanzette dienen. In Bezug auf die Drehachse 19 erstreckt sich die Kammer 5 in radialer Richtung. Die auf dem Stechschlitten 21 montierte Treibstange 9 befindet sich hier noch vollständig außerhalb der Kammer 5, kann aber zur Ausführung eines Stiches von hinten in die Kammer 5 eindringen, um an die dort gelagerte Lanzette anzukoppeln und diese in radialer Richtung nach vorne in Richtung der Öffnung 8 zu treiben.

Gut zu erkennen in Figur 6 ist die Funktion des Druckstößels 37, der durch eine Eintrittsöffnung 42 von unten in die Kammer 5 eindringen kann, um die Lanzette nach dem Stich in Kontakt mit einem Testelement zu bringen. Im Schnitt ist ferner die Antriebsfeder 43 zu sehen, die zwischen Spannrotor 18 und Antriebsrotor 20 sitzt.

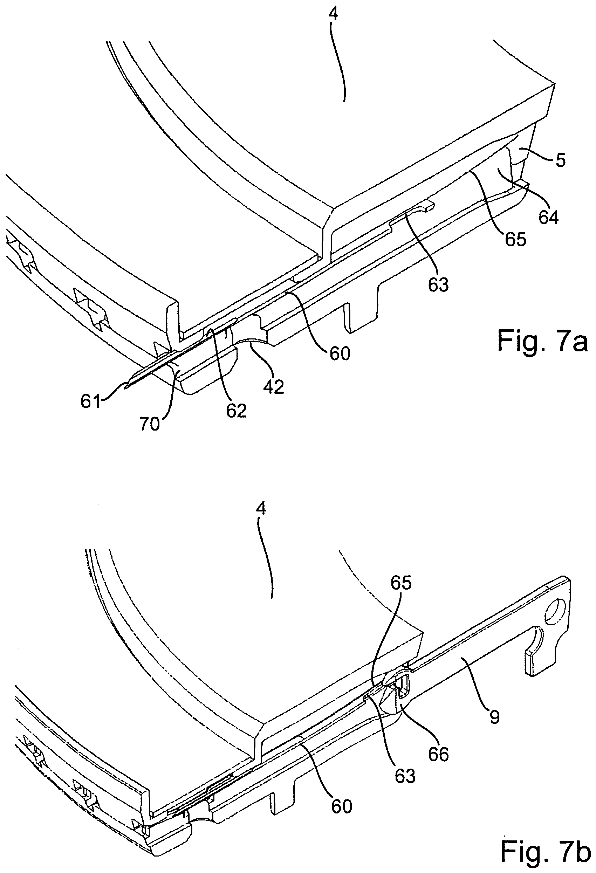

In Figur 7a befindet sich eine Lanzette 60 teilweise in der Kammer 5. Die Lanzette 60 ist aus einem Stück ebenem flachen Blech gefertigt, das um eine quer zur Stichachse verlaufende Biegeachse elastisch biegbar ist. Da der Schnitt in Figur 7 entlang der Längsachse der Lanzette 60 verläuft, ist nur eine Hälfte der Lanzette 60 zu sehen. Die andere Hälfte ist spiegelbildlich ausgebildet. Vorne ist die Lanzette 60 scharf geschliffen und bildet eine Spitze 61, an die sich eine Kapillarrinne 62 zur Aufnahme der Blutprobe anschließt. Im Bereich ihres hinteren Endes weist die Lanzette 60 eine Öse 63 auf, die der Ankopplung der Treibstange 9 (vgl. Fig. 6) dient.

Die Kammer 5 hat einen Schacht 64, der dem Querschnitt der Lanzette 60 angepasst ist. Bei diesem Ausführungsbeispiel, bei dem die Lanzette aus einem ebenen flachen Blech gefertigt ist, hat der Schacht 64 im Wesentlichen einen rechteckigen Querschnitt mit einer Höhe, die minimal größer ist als die Dicke der Lanzette 60, die mit etwas Spiel im Schacht 64 hin- und hergleiten kann. In ihrem hinteren, radial nach innen weisenden Bereich weist der Schacht 64 eine Krümmung 65 auf, die sich entlang der Stichachse erstreckt. Figur 7b verdeutlicht das Zusammenspiel zwischen der Kammer 5 mit gekrümmtem Schacht 64, der elastisch gebogenen Lanzette 60 und der Treibstange 9, die hier gerade in das hintere Ende der Kammer 5 einfährt.

In Figur 7a ist die Lanzette 60 noch in ebenem entspanntem Zustand zu sehen. Wird die Lanzette 60 nun, ausgehend von dieser Lage, in radialer Richtung nach innen geschoben, muss sie der Krümmung 65 des Schachtes 64 folgen und sich dabei elastisch verbiegen. Gleichzeitig verlagert sich die Öse 63 vertikal nach oben. Wird umgekehrt die Lanzette 60 radial nach außen geschoben, verlagert sich die Öse 63 wieder vertikal nach unten. Diese Verlagerung lässt sich zum An- und Abkoppeln der Treibstange 9 benutzen, wozu die Treibstange 9 eine entsprechende Kopplungsstruktur aufweist.

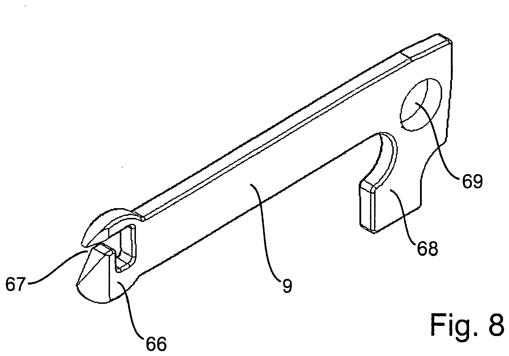

Gemäß Figur 8 weist die Treibstange 9 an ihrem vorderen Ende einen Haken 66 auf, der in die Öse 63 der Lanzette 60 (vgl. Fig. 7a) einhakbar ist. Der Schlitz 67 im Haken 66 ist gerade so groß, dass das hintere Ende der Lanzette 60 hindurchpasst. An ihrem in Stichrichtung hinteren Ende weist die Treibstange 9 einen Schaft 68 mit einem Befestigungsloch 69 auf. Damit lässt sich die Treibstange auf den Stechschlitten 21 montieren (vgl. Figuren 2 und 6).

Die Treibstange 9 ist aus einem einzigen Stück Stahlblech gefertigt. Die Treibstange 9 ist erheblich schmaler als die Lanzette 60. Zur Führung der Treibstange 9 in der Kammer 5 weist deren Schacht 64 eine zentrale Führungsnut 70 auf, deren Höhe und Breite den Abmessungen der Treibstange 9 angepasst sind (vgl. Fig. 7b). Dadurch kann die relativ schmale Treibstange 9 in der Mitte des Schachts 64 laufen, ohne dass die Treibstange 9 der Krümmung 65 folgen muss, welche nur links und rechts an der Lanzette 60 angreift. Die elastisch biegbare Lanzette 60 mit Öse 63, die Kammer 5 mit Schacht 64 und Krümmung 65 sowie die Treibstange 9 mit Haken 66 bilden einen Lanzettenkopplungsmechanismus, bei dem die Lanzette 60 selbsttätig an die Treibstange 9 ankoppelt, wenn die Treibstange 9 in die Kammer 4 eindringt und die Lanzette 60 radial nach außen treibt.

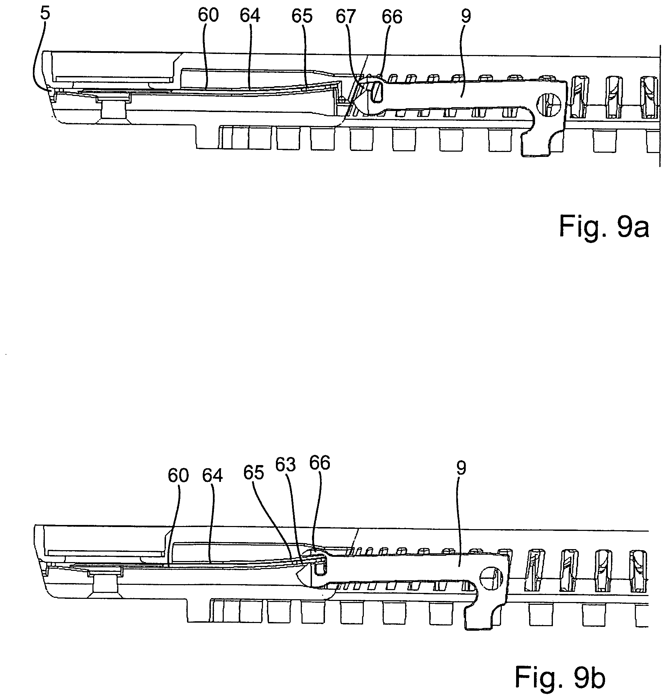

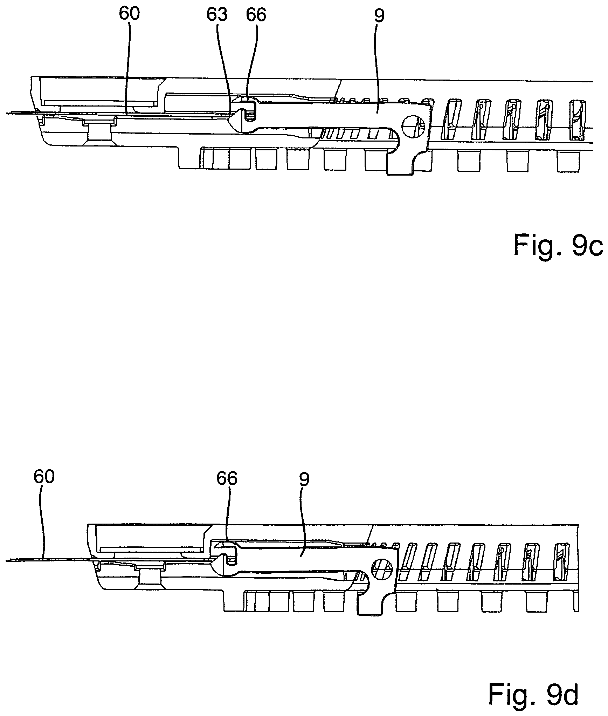

Die Ankopplung der Treibstange 9 an die Lanzette 64 in der Vorbereitungsphase eines Stiches wird nun anhand der Figuren 9a, 9b, 9c und 9d beschrieben:
In Figur 9a befindet sich die Treibstange 9 noch außerhalb der Kammer 5. Die Lanzette 60 befindet sich vollständig in dem Schacht 64. Der hintere Bereich der Lanzette 60 wird durch die Krümmung 65 senkrecht aus der Blechebene heraus gebogen, in der Abbildung nach oben. Damit überragt das hintere Ende der Lanzette 60 den Schlitz 67 des Hakens 66 der Treibstange 9.

In Figur 9b hat sich die Treibstange 9 ein kleines Stück in radialer Richtung nach außen (in der Abbildung nach links) bewegt, wobei sie von hinten in die Kammer 5 eingedrungen ist. Das hintere Ende der Lanzette 60 ist durch den Schlitz 67 des Hakens 66 hindurchgerutscht, so dass der Haken 66 nun in die Öse 63 von unten eingreifen kann. Das Einhaken der Öse 63 wird dabei unterstützt durch die Federspannung der elastisch verbogenen Lanzette 60.

Dringt nun die Lanzette 9 weiter in die Kammer 5 ein, so treibt sie die Lanzette 60 weiter in radialer Richtung nach außen aus der Kammer 5 hinaus. Dies ist die schnelle Stichbewegung zur Erzeugung einer kleinen Wunde.

In Figur 9c hat die Lanzette 60 den Bereich der Krümmung 65 (vgl. Fig. 9b) verlassen. Die Lanzette 60 ist nun in entspanntem Zustand und der Haken 66 ist vollständig in die Öse 63 eingehakt.

In Figur 9d hat die Lanzette 60 ihren Umkehrpunkt erreicht. Dies entspricht der maximalen Einstechtiefe. Die Bewegungsrichtung der Treibstange 9 wird nun umgekehrt. Die Hinterschneidung des Hakens 66 erlaubt es, dass die Treibstange 9 die Lanzette 60 nicht nur nach vorne drücken, sondern auch nach hinten ziehen kann, wobei die formschlüssige Verbindung zwischen Haken 66 und Öse 63 dabei erhalten bleibt.
Im Zuge des weiteren Rückzugs der Treibstange 9 und der angehängten Lanzette 60 erfolgt schließlich ein automatisches Abkoppeln der Lanzette beim Einfahren in den Bereich der Krümmung 65. Der Vorgang des Abkoppelns entspricht dabei genau dem beschriebenen Ankoppeln, jedoch in umgekehrter Richtung. Figur 9b entspricht dabei dem Moment, in dem der Haken 66 die Öse 63 wieder freigibt.

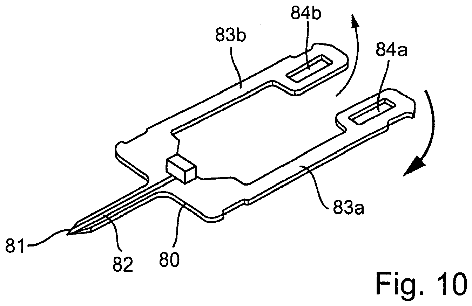

Die alternative Lanzette 80 gemäß Fig. 10 hat ebenfalls eine Spitze 81 und eine sich daran anschließende Kapillarrinne 82. Im Unterschied zu der zuvor beschriebenen Lanzette teilt sich der Körper der Lanzette 80 in zwei Arme 83a und 83b, die spiegelbildlich ausgebildet sind. Im Bereich des Endes ist jeweils eine Öse 84a bzw. 84b vorgesehen, die zum Ankoppeln an eine mit zwei parallelen Haken ausgestattete Treibstange dienen. Auch diese Lanzette 80 ist aus einem Stück ebenen Blechs hergestellt, das elastisch verbiegbar ist. Der eine Arm 83a lässt sich um eine quer zur Stichachse verlaufende Biegeachse elastisch nach oben biegen, während der andere Arm 83b in die entgegengesetzte Richtung nach unten verbogen werden kann. Zur Lagerung der Lanzette 80 in einem Magazin müssen die Magazinkammern jeweils einen rechten und einen linken Schacht umfassen, wobei der eine Schacht nach oben und der andere Schacht nach unten gekrümmt ist.

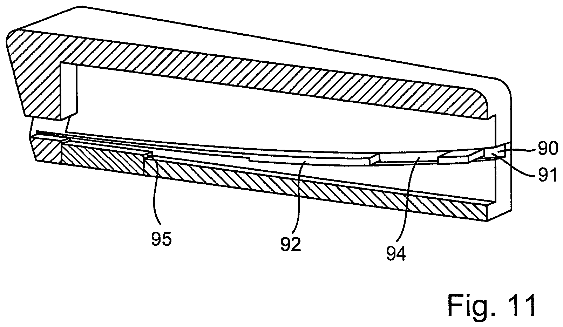

Die in Fig. 11 dargestellte alternative Ausführung eines Lanzettenkopplungsmechanismus weist eine Magazinkammer 90 mit einem Schacht 91 auf, der über seine gesamte Länge um eine Achse quer zur Stichrichtung gekrümmt ist. Entsprechend wird die in den Schacht 91 eingeführte Lanzette 92 auf ihrer gesamten Länge und damit auch in ihrem vorderen Bereich (in der Abbildung links) elastisch verbogen. Zur An- und Abkopplung der Lanzette 92 an eine Treibstange dient wiederum eine Öse 94, die nahe des hinteren Endes der Lanzette 92 vorgesehen ist und sich bei einer Bewegung der Lanzette 92 in Stichrichtung (in der Abbildung nach links) entsprechend der Krümmung des Schachts 91 nach unten bewegt. Aufgrund der Biegespannung wird somit die Spitze 93 der Lanzette 92 nach unten gedrückt und trifft dort auf ein Testfeld 94, das unmittelbar unterhalb des Schachts 91 angeordnet ist. Auf diese Weise kann das von der Lanzette 92 während eines Stechvorgangs aufgenommene Blut automatisch auf das Testfeld 95 übertragen werden, ohne dass es hierzu weiterer Hilfsmittel bedarf.

### Liste der Bezugszeichen

- 1: Grundplatte
- 2: Abdeckung
- 3: Aufnahme
- 4: Magazin
- 5: Kammer (von 4)
- 6: Andruckbügel
- 7: Fixierring (an 6)
- 8: Öffnung (in 6)
- 9: Treibstange
- 10: Austrittsöffnung (in 2)
- 11: Kulissenschieber
- 12: Probenübertragungseinrichtung
- 13: Lanzette

- 15: Elektromotor
- 16: Schneckenwelle
- 17: Schneckenrad
- 18: Spannrotor
- 19: Drehachse
- 20: Antriebsrotor
- 21: Stechschlitten
- 22: Übersetzungshebel
- 23: Hebelschlitten
- 24: Langloch (in 22)
- 25: Drehzapfen (an 21)
- 26: Gabel (von 22)
- 27: Zapfen
- 28: Nutenreiter
- 29: Steuernut (in 20)
- 30: Zahnstange (an 22)
- 31: Zahnrad
- 32: Zahnritzel
- 33: Führung
- 34: Schaltkulisse (von 11)
- 35: Schaltnocke (an 18)
- 36: Federbügel
- 37: Druckstößel (von 12)
- 38: Gleithülse
- 39: Gleitrolle
- 40: Rampe (an 18)
- 41: Federbügel
- 42: Eintrittsöffnung (von 5)
- 43: Antriebsfeder

- 45: Zahnkranz
- 46: Ausnehmung
- 47: Sperrklinke (an 18)
- 48: Sperrzapfen (18)
- 49: Nut (in 20)
- 50: Aussparung (in 20)
- 51: Gleitzapfen (von 11)

- 60: Lanzette
- 61: Spitze
- 62: Kapillarrinne
- 63: Öse
- 64: Schacht (von 5)
- 65: Krümmung (von 64)
- 66: Haken (an 9)
- 67: Schlitz
- 68: Schaft
- 69: Befestigungsloch
- 70: Führungsnut (von 64)

- 80: Lanzette
- 81: Spitze
- 82: Kapillarrinne
- 83a, 83b: Arme
- 84a, 84b: Ösen

- 90: Magazinkammer
- 91: Schacht (von 90)
- 92: Lanzette
- 93: Spitze
- 94: Öse
- 95: Testfeld

## Patentansprüche

1. Lanzettenkopplungsmechanismus für einen Apparat zur Gewinnung und Analyse einer Blutprobe, mit wenigstens einer Kammer, die sich in Richtung einer Stichachse erstreckt und eine Lanzette enthält, und mit einer Treibstange, die in die Kammer eindringt und an die Lanzette ankoppelbar ist, um eine kontrollierte Vor- und Rückbewegung entlang der Stichachse auszuführen, wobei die Lanzette (60) um wenigstens eine quer zur Stichachse verlaufende Biegeachse elastisch biegbar ist; die Kammer (5) einen dem Querschnitt der Lanzette (60) angepassten Schacht (64) umfasst, der eine zentrale Führungsnut (70) aufweist, deren Höhe und Breite der Abmessungen der Treibstange (9) angepasst ist; die Treibstange (9) mit der Lanzette in formschlüssiger Verbindung steht, wenn sich die Lanzette in entspanntem Zustand befindet
**gekennzeichnet dadurch, dass**
der Schacht (64) zumindest in seinem hinteren Bereich mindestens eine sich entlang der Stichachse erstreckende Krümmung um eine Achse quer zur Stichachse aufweist und
die Treibstange an die Lanzette ankoppelbar ist, wenn sich die Lanzette in entsprechend der Krümmung der Kammer gebogenem Zustand befindet.

2. Lanzettenkopplungsmechanismus nach Anspruch 1, **dadurch gekennzeichnet, dass**
die Lanzette (60) mindestens eine Ankoppelausnehmung aufweist;
die Treibstange (9) an ihrem vorderen Ende wenigstens eine Kopplungsstruktur aufweist, die sich senkrecht zur Biegeachse der Lanzette (60) erstreckt und in die Ankoppelausnehmung der gebogenen Lanzette (60) eingreift, wenn die Lanzette (60) sich vorwärts bewegt.

3. Lanzettenkopplungsmechanismus nach Anspruch 2, **dadurch gekennzeichnet, dass**
die Lanzette (60) aus einem Stück ebenem flachen Blech gefertigt ist und im Bereich ihres hinteren Endes wenigstens eine Öse (63) aufweist;
die Treibstange (9) an ihrem vorderen Ende wenigstens einen Haken (66) aufweist, der in die Öse (63) einhakbar ist.

4. Lanzettenkopplungsmechanismus nach Anspruch 3, **dadurch gekennzeichnet, dass** der Schacht (64) einen im Wesentlichen rechteckigen Querschnitt hat, dessen Höhe minimal größer ist als die Dicke der Lanzette (60), so dass die Lanzette (60) mit etwas Spiel im Schacht (64) hin- und hergleiten kann.

5. Lanzettenkopplungsmechanismus nach einem der vorherigen Ansprüche, **dadurch gekennzeichnet, dass** die Treibstange (9) aus einem einzigen Stück Stahlblech gefertigt ist.

6. Lanzettenkopplungsmechanismus nach einem der vorherigen Ansprüche, **dadurch gekennzeichnet, dass** die Lanzette (80) zwei spiegelbildlich ausgebildete Arme (83a, 83b) hat, an deren Enden jeweils eine Öse (84a, 84b) vorgesehen ist.

7. Lanzettenkopplungsmechanismus nach Anspruch 6, **dadurch gekennzeichnet, dass** der eine Arm (83a) nach oben und der andere Arm (83b) nach unten elastisch biegbar ist.

8. Lanzettenkopplungsmechanismus nach Anspruch 6 oder 7, **dadurch gekennzeichnet, dass** die Kammer einen rechten und einen linken Schacht umfasst, wobei der eine Schacht nach oben und der andere Schacht nach unten gekrümmt ist.

9. Lanzettenkopplungsmechanismus nach einem der Ansprüche 1 bis 5, **dadurch gekennzeichnet, dass** der Schacht (91) über seine gesamte Länge um eine Achse quer zur Stichachse gekrümmt ist, so dass die in dem Schacht (91) eingeführte Lanzette (92) auf ihrer gesamten Länge elastisch verbogen ist.

## Claims

1. Lancet coupling mechanism for an apparatus for obtaining and analyzing a blood sample,
having at least one chamber, which extends in the direction of a piercing axis and contains a lancet,
and having a drive rod, which penetrates into the chamber and can be coupled onto the lancet, in order to execute a controlled forward and reverse movement along the piercing axis,
wherein
the lancet (60) is elastically bendable around at least one bending axis extending transversely to the piercing axis;
the chamber (5) comprises a shaft (64), which is adapted to the cross section of the lancet (60) and has a central guide groove (70), whose height and width are adapted to the dimensions of the drive rod (9);
the drive rod (9) has a formfitting connection to the lancet when the lancet is in the relaxed state,
**characterized in that**
the shaft (64) at least in its rear area has at least one curvature around an axis transverse to the piercing axis, said curvature extending at least along the piercing axis, and
the drive rod can be coupled onto the lancet when the lancet is in the bent state in accordance with the curvature of the chamber.

2. Lancet coupling mechanism according to claim 1, **characterized in that**
the lancet (60) has at least one coupling recess;
the drive rod (9) on its front end has at least one coupling structure, which extends perpendicularly to the bending axis of the lancet (60) and
engages in the coupling recess of the bent lancet (60) when the lancet (60) moves forward.

3. Lancet coupling mechanism according to claim 2, **characterized in that**
the lancet (60) is manufactured from a piece of level flat sheet metal and in the area of its rear end has at least one eye (63);
the drive rod (9) on its front end has at least one hook (66), which can be hooked into the eye (63).

4. Lancet coupling mechanism according to claim 3, **characterized in that**
the shaft (64) essentially has a rectangular cross section having a height which is minimally greater than the thickness of the lancet (60), which is slidable back and forth with some play in the shaft (64).

5. Lancet coupling mechanism according to any one of the preceding claims, **characterized in that** the drive rod (9) is manufactured from a single piece of sheet steel.

6. Lancet coupling mechanism according to any one of the preceding claims, **characterized in that** the lancet (80) comprises two arms (83a, 83b), which are implemented as mirror images, and **in that** eyes (84a, 84b) are provided at the end of the arms (83a, 83b), respectively.

7. Lancet coupling mechanism according to claim 6, **characterized in that** the one arm (83a) is bendable elastically upward, while the other arm (83b) is bendable elastically downwards.

8. Lancet coupling mechanism according to claim 6 or 7, **characterized in that** the chamber comprises a right shaft and a left shaft, one shaft being curved upward and the other shaft being curved downward.

9. Lancet coupling mechanism according to one of the claims 1 to 5, **characterized in that** the shaft (91) is curved over its entire length around an axis transverse to the piercing axis so that the lancet (92) inserted into said shaft (91) is elastically bent on its entire length.

## Revendications

1. Mécanisme de couplage de lancette pour un appareil d'obtention et d'analyse d'un échantillon sanguin,
avec au moins une chambre qui s'étend en direction d'un axe de piqûre et qui contient une lancette, et
avec une bielle motrice qui pénètre dans la chambre et peut être couplée à la lancette pour mettre en oeuvre un mouvement commandé vers l'avant et vers l'arrière le long de l'axe de piqûre,
dans lequel
la lancette (60) peut être fléchie de manière élastique autour d'au moins un axe de flexion passant transversalement par rapport à l'axe de piqûre ;
la chambre (5) comprend une goulotte (64) adaptée à la section de la lancette (60), ladite goulotte présentant une rainure de guidage centrale (70) dont la hauteur et la largeur sont adaptées aux dimensions de la bielle motrice (9) ;
la bielle motrice (9) est en liaison par complémentarité de forme avec la lancette, lorsque la lancette se trouve dans un état déployé,
**caractérisé en ce que**
la goulotte (64) présente au moins dans son secteur postérieur au moins un cintrage s'étendant le long de l'axe de piqûre autour d'un axe perpendiculaire à l'axe de piqûre et
la bielle motrice peut être couplée à la lancette lorsque la lancette se trouve dans un état fléchi correspondant au cintrage de la chambre.

2. Mécanisme de couplage de lancette selon la revendication 1, **caractérisé en ce que**
la lancette (60) présente au moins un évidement de couplage ;
la bielle motrice (9) présente au niveau de son extrémité antérieure au moins une structure de couplage qui s'étend perpendiculairement à l'axe de flexion de la lancette (60) et qui engrène dans l'évidement de couplage de la lancette (60) fléchie lorsque la lancette (60) se déplace vers l'avant.

3. Mécanisme de couplage de lancette selon la revendication 2, **caractérisé en ce que**
la lancette (60) est réalisée à partir d'une pièce de tôle plane lisse et présente au moins un oeillet (63) dans le secteur de son extrémité postérieure ;
la bielle motrice (9) présente au niveau de son extrémité antérieure au moins un crochet (66) qui peut être accroché dans l'oeillet (63).

4. Mécanisme de couplage de lancette selon la revendication 3, **caractérisé en ce que**
la goulotte (64) possède une section essentiellement rectangulaire dont la hauteur est au moins supérieure à l'épaisseur de la lancette (60), de sorte que la lancette (60) peut glisser d'un bout à l'autre de la goulotte (64) avec un peu de jeu.

5. Mécanisme de couplage de lancette selon l'une quelconque des revendications précédentes, **caractérisé en ce que** la bielle motrice (9) est réalisée à partir d'une pièce unique de tôle d'acier.

6. Mécanisme de couplage de lancette selon l'une quelconque des revendications précédentes, **caractérisé en ce que** la lancette (80) possède deux bras (83a, 83b) réalisés de manière spéculaire, au niveau des extrémités desquels est prévu respectivement un oeillet (84a, 84b).

7. Mécanisme de couplage de lancette selon la revendication 6, **caractérisé en ce que** l'un des bras (83a) peut être fléchi de manière élastique vers le haut et l'autre bras (83b) peut être fléchi de manière élastique vers le bas.

8. Mécanisme de couplage de lancette selon la revendication 6 ou 7, **caractérisé en ce que** la chambre comprend une goulotte droite et une goulotte gauche, dans lequel l'une des goulottes est cintrée vers le haut et l'autre goulotte est cintrée vers le bas.

9. Mécanisme de couplage de lancette selon l'une quelconque des revendications 1 à 5, **caractérisé en ce que** la goulotte (91) est cintrée sur toute sa longueur autour d'un axe perpendiculairement à l'axe de piqûre, de sorte que la lancette (92) introduite dans la goulotte (91) est incurvée de manière élastique sur toute sa longueur.
